# EUROPÄISCHE PATENTSCHRIFT

(11) **EP 1 051 122 B1**
(45) Veröffentlichungstag und Bekanntmachung des Hinweises auf die Patenterteilung: **27.11.2002**
(21) Anmeldenummer: 98900521.0
(22) Anmeldetag: 27.01.1998
(51) Int. Cl.: A61B 19/00

(54) **VORRICHTUNG ZUR KALIBRIERUNG UND VERIFIZIERUNG DER GENAUIGKEIT VON CHIRURGISCHEN INSTRUMENTEN**
DEVICE FOR GAUGING AND VERIFYING THE PRECISION OF SURGICAL INSTRUMENTS
DISPOSITIF DE CALIBRAGE ET DE VERIFICATION DE LA PRECISION D'INSTRUMENTS CHIRURGICAUX

(43) Veröffentlichungstag der Anmeldung: 15.11.2000
(73) Patentinhaber: SYNTHES AG CHUR, 7002 Chur (CH); SYNTHES (U.S.A.), Paoli, PA 19301-1222 (US)
(72) Erfinder: FORRER, Ruth, CH-4313 Möhlin (CH); SCHERRER, José, L., CH-4702 Oensingen (CH)
(74) Vertreter: Lusuardi, Werther Giovanni, Dr.
(86) Internationale Anmeldenummer: CH9800030
(87) Internationale Veröffentlichungsnummer: WO99037232

(56) Entgegenhaltungen:
- WO-A-97/29683
- WO-A-97/29710
- US-A- 5 323 543
- US-A- 5 483 961
- US-A- 5 617 857

## Beschreibung

Die Erfindung bezieht sich auf eine Vorrichtung zur Kalibrierung und Verifizierung der Genauigkeit von chirurgischen Instrumenten gemäss dem Oberbegriff des Patentanspruchs 1.

Seit dem grundlegenden durch die digitalisierte Bilddarstellung (Ultraschalltechnik, Computer Tomography (CT scan) Magnetic Resonance Imaging (MRI)) verursachten Wechsel in der Medizinischen Wissenschaft hat sich die dreidimensionale Bildverarbeitung getragen durch diese Erfassungssysteme zunehmend entwickelt und als Anwendung in der Orthopädie und Traumatologie eingebürgert. Dreidimensionale Bilder, welche die Oberfläche und das Volumen von Knochen und Gelenken sichtbar darstellen, werden durch moderne Computer einfach erstellt und geben dem Chirurgen einen Überblick über die Verletzung des Knochens oder Gelenkes wie diese während des Eingriffes zu sehen wäre. Diese dreidimensionalen Systeme öffnen den Weg zur Simulation von chirurgischen Eingriffen, virtueller Manipulation von knöchernen Teilen, anatomischer Modellierung und an Knochen und Gelenke angepassten Prothesen. Die Hauptanwendungsgebiete der dreidimensionalen Bildverarbeitungstechnik liegen in der Simulation vor dem operativen Eingriff und der bildgeführten Navigation der chirurgischen Instrumente am Patienten während das Eingriffes. Die Navigation während eines chirurgischen Eingriffes verlangt das präzise Erfassen der Position und Orientierung von chirurgischen Instrumenten hinsichtlich eines räumlichen Bezugssystemes. Die Navigation während des operativen Eingriffes eröffnet dem Benützer eines solchen dreidimensionalen Systems folgende Möglichkeiten:
- Während der Operation werden chirurgische Instrumente in real time Darstellung auf dem Bildschirm gezeigt. Der Chirurg kann jederzeit genau beobachten wo sich das Instrument momentan am Patienten befindet.
- Ein Softwaremodul zur realtime Erstellung von Trajektorien zeigt am Bildschirm abhängig von der gegenwärtigen Instrumentenorientierung den beabsichtigten Pfad für das Instrument. Dies gestattet eine genaue Positionierung des Instrumentes ohne voroperative Planung.
- Ein Guidance Softwaremodul ermöglicht dem Chirurgen, den vor der Operation geplanten Instrumentenweg mit der gegenwärtigen Position des Instrumentes und dem aus dieser gegenwärtigen Position und Orientierung des Instrumentes resultierenden Weg zu vergleichen, wobei der präoperativ geplante Pfad in intraoperative Instrumentenführung umgesetzt werden kann.

Dabei können übliche chirurgische Instrumente (Bohrer, Löffel, Scheren, Pinzetten, Sonden etc.) verwendet werden. Zur Positionserfassung mittels eines dreidimensionalen Koordinatenmesssystems müssen die chirurgischen Instrumente mit Markern oder Sensoren zur Abgabe oder zum Empfang von elektromagnetischen Wellen, akustischen Wellen oder magnetischer Felder ausgestattet sein. Solche Koordinatenmesssysteme sind oft mit Problemen behaftet. Bei Ultraschallmessungen beruhen diese Probleme auf der Tatsache, dass für die Ultraschallübertragung das Medium Luft benötigt wird. Die physikalischen Eigenschaften der Luft ändern sich mit den äusseren Parametern Temperatur, Druck und Luftfeuchtigkeit zum Teil sehr stark. Die äusseren Parameter müssen ständig gemessen werden, um die nötigen Daten zu erhalten, damit die Abweichungen fortlaufend kompensiert werden können. Sonst resultiert ein grösserer Fehler in der Positionsbestimmung. Im Falle der Magnetfeldvermessung müssen auch hier die äusseren Parameter wie
- Störfelder (ausgehend von z.B. Bildschirmen, Computern oder Elektromotoren); und
- Nichtpermeable Stoffe im Magnetfeld (z.B. Metalle, die im Magnetfeld bewegt werden).

Eine Vorrichtung mit einem fest zu einem Operationstisch ausrichtbaren Rahmen, welcher Kalibrieraufnahmen für eine chirurgische Sonde enthält, ist aus der WO 97/29683 ACKER bekannt. Diese bekannte Vorrichtung umfasst eine medizinische Sonde mit daran befestigten Markern, einen relativ zum Patienten fixierbaren Rahmen mit daran befestigten Markern, Übertragungsmittel zur Übertragung beispielsweise eines magnetischen Feldes zwischen den Markern an der Sonde und denjenigen am Rahmen, Messfühler zur Erfassung jedes dieser Felder und eine Recheneinheit zur Bestimmung der relativen Position der Sonde bezüglich der Marker am Rahmen aus den Daten der erfassten Felder. Die Kalibrieraufnahmen am Rahmen gestatten nun, vor dem Einsatz der Sonde am menschlichen Körper, die Sonde mit ihrer Spitze in jede Kalibrieraufnahme zu führen und einen Vergleich zwischen den bekannten Positionen der Kalibrieraufnahmen mit den gemessenen Positionen der Sonde durchzuführen.

Nachteil dieser bekannten Vorrichtung ist, dass die Kalibrieraufnahmen lediglich für eine einzige Sonde verwendbar sind.

Hier will die Erfindung Abhilfe schaffen. Der Erfindung liegt die Aufgabe zugrunde, die werkseitige Kalibrierung zu überprüfen, um Deformationen respektive Abnützungen der chirurgischen instrumente festzustellen. Bei Nadeln oder Bohrern soll die unterschiedliche Länge des Einsatzes eruiert werden.

Die Erfindung löst die gestellte Aufgabe mit einer Vorrichtung zur exakten Kalibrierung und Verifizierung der Genauigkeit von chirurgischen Instrumenten, welche die Merkmale des Anspruchs 1 aufweist.

In einer bevorzugten Ausführungsform der erfindungsgemässen Vorrichtung umfasst diese eine frei positionierbare Halterung, ein in der Halterung gelagertes und darin bewegbares Element, sowie ein Mittel zur exakten Führung des bewegbaren Elementes in der Halterung. Das bewegbare Element ist mit mehreren zur Vorrichtung genau ausrichtbaren Hohlräumen versehen. Jeder Hohlraum ist so beschaffen, dass seine Gestalt zu einem bestimmten chirurgischen Instrument exakt korrespondiert. Das im entsprechenden Hohlraum aufgenommene chirurgische Instrument wird daher in einer zur Vorrichtung definierten Position und Orientierung aufgenommen. Das Führungsmittel, welches eine exakte Bewegung des bewegbaren Elementes in der Halterung gewährleistet, kann zum Einbringen oder Entfernen des bewegbaren Elementen aus der Halterung entfernt werden. Damit ist ermöglicht, dass ein bewegbares Element gegen ein anderes mit anderen Hohlräumen versehenes bewegbares Element ausgetauscht werden kann. Jedes bewegbare Element ist an seiner äusseren Mantelfläche mit Vertiefungen versehen, die so zu den Hohlräumen korrespondieren, dass ein in die Vertiefungen einrastendes Mittel, das an der Halterung angebracht ist, in eine der Vertiefungen einrastet und damit den korrespondierenden Hohlraum in einer bezüglich der Position und Orientierung der Vorrichtung genau definierten Lage lösbar fixiert. Die Vorrichtung ist mit sechs Light Emitting Dioden (LED) versehen. Damit kann die Position und die Orientierung der Vorrichtung durch eine Vermessungseinrichtung genau ermittelt werden. Die Vermessung der Position und Orientierung der Vorrichtung mittels den von den LED abgegebenen elektromagnetischen Wellen kann durch Laufzeitmessung, Interferometrie oder Videogrammetrie erfolgen. Als Vermessungseinrichtung ist beispielsweise ein im Handel erhältliches dreidimensionales Bewegungsmesssystem OPTOTRAK 3020 von Northern Digital Inc. denkbar. Ein die Position und Orientierung der Vorrichtung festhaltender, löschbarer elektronischer Datenspeicher kann in der Computersoftware einprogrammiert oder in der Computerhardware enthalten sein.

Eine andere Ausführungsform der erfindungsgemässen Vorrichtung besteht darin, dass als einziger Unterschied zur vorangehend besprochenen Ausführungsform das bewegbare Element ein Zylinder mit einer Zentralachse und einer koaxialen durchgehenden Bohrung ist. Als führendes Mittel ist ein in der Vorrichtung lösbar angebrachter durch die Bohrung im bewegbaren Element und in der Halterung durchgehender Bolzen eingesetzt. Der Bolzen passt praktisch ohne Spiel in die Bohrung, wodurch die Bewegung des bewegbaren Elementes, die hier einer Rotation entspricht, präzis um die Zentralachse verläuft. Der Bolzen ist an seinem einen Ende mit einem Gewinde versehen, das in eine untere Platte der Halterung eingeschraubt werden kann. Die lösbare Verbindung des Bolzens mit der Halterung ermöglicht den Austausch des bewegbaren Elementes. An seinem anderen Ende ist der Bolzen mit Mitteln versehen, die ein einfaches Ein- beziehungsweise Ausschrauben des Bolzens aus der Vorrichtung ermöglichen.

Wiederum eine andere Ausführungsform der erfindungsgemässen Vorrichtung unterscheidet sich von der vorangehend beschriebenen Ausführungsform nur dadurch, dass das an der Halterung angebrachte, in die Vertiefungen am bewegbaren Element einrastende Mittel ein Federdruckteil ist. Dieses Federdruckteil ist in ein entsprechend in der Halterung vorgesehenes Gewinde eingeschraubt. Es besteht im wesentlichen aus einer Zylinderschraube, einer Druckfeder und einer Kugel oder einem vorne angeschrägten Stift. Kugel oder Stift und Feder sind in der Zylinderschraube axial zusammenpressbar eingelassen, so dass die Kugel oder der Stift durch die Federkraft in die Vertiefungen einrastet.

Eine weitere Ausführungsform der erfindungsgemässen Vorrichtung unterscheidet sich von der vorhergehend beschriebenen Ausführungsform nur dadurch, dass die Mittel, die die Bestimmung der Position und Orientierung der Vorrichtung durch eine Vermessungseinrichtung ermöglichen, elektromagnetische Wellen emittierende Sender sind.

Eine andere Ausführungsform der erfindungsgemässen Vorrichtung unterscheidet sich von den oben beschriebenen Varianten nur dadurch, dass die Mittel, die die Bestimmung der Position und Orientierung der Vorrichtung durch eine Vermessungseinrichtung ermöglichen, optische Leuchtquellen sind. Anstelle der optischen Leuchtquellen können natürlich auch optische Reflektoren, die von im Raum befindlichen Lichtquellen beleuchtet werden, Infrared Light Emitting Dioden (IRED) oder durch eine Lichtquelle gespiesene optische Faserleiter eingesetzt werden.

Wiederum eine andere Ausführungsform der erfindungsgemässen Vorrichtung unterscheidet sich von den vorangehend beschriebenen Varianten dadurch, dass die Vermessungseinrichtung auf der Basis von akustischen Wellen arbeitet. Die an der Vorrichtung angebrachten Mittel, die die Bestimmung der Position und Orientierung der Vorrichtung durch die Vermessungseinrichtung ermöglichen, sind akustische Wellen emittierende Sender.

Eine weitere Ausführungsform der erfindungsgemässen Vorrichtung unterscheidet sich von der vorher beschriebenen dadurch, dass die Mittel, die die Bestimmung der Position und Orientierung der Vorrichtung durch die Vermessungseinrichtung ermöglichen, Mikrophone sind.

Wiederum eine andere Ausführungsform der erfindungsgemässen Vorrichtung unterscheidet sich von den vorangehend beschriebenen Variante dadurch, dass die Vermessungseinrichtung auf der Basis von magnetischer Induktion arbeitet. Die an der Vorrichtung angebrachten Mittel, die die Bestimmung der Position und Orientierung der Vorrichtung durch die Vermessungseinrichtung ermöglichen, sind Induktionsspulen.

Eine andere Ausführungsform der erfindungsgemässen Vorrichtung unterscheidet sich von der vorangehend beschriebenen Variante dadurch, dass die Mittel, die die Bestimmung der Position und Orientierung der Vorrichtung durch die Vermessungseinrichtung ermöglichen, Hall-Sensoren sind.

Eine weitere Ausführungsform der erfindungsgemässen Vorrichtung kann nach einem der oben beschriebenen Vermessungsverfahren bezüglich Position und Orientierung ausgerüstet sein, unterscheidet sich aber dadurch von den oben beschriebenen Ausführungsformen, dass der die Position und Orientierung der Vorrichtung festhaltende, löschbare elektronische Datenspeicher ein EPROM (Erasable Programmabel Read-Only Memory) ist, das im Stecker am Verbindungskabel zu dem Computer untergebracht ist.

Die durch die Erfindung erreichten Vorteile sind im wesentlichen darin zu sehen, dass dank der erfindungsgemässen Vorrichtung verschiedene chirurgische Instrumente rasch bezüglich ihrer Position und Orientierung im Raum kalibriert werden können. Dies ermöglicht, dass Vermessungsfehler sofort korrigiert werden können. Diese Fehlerkorrektur ist jederzeit auch während einer Operation denkbar. Zudem können die chirurgischen Instrumente auch exakt bezüglich Position und Orientierung kalibriert werden, wenn die Gestalt der Instrumente keinen ausgeprägten Punkt oder eine Spitze aufweist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Explosionsdarstellung einer Ausführungsform der erfindungsgemässen Vorrichtung; und
Fig. 2 einen Schnitt durch eine Ausführungsform der erfindungsgemässen Vorrichtung.

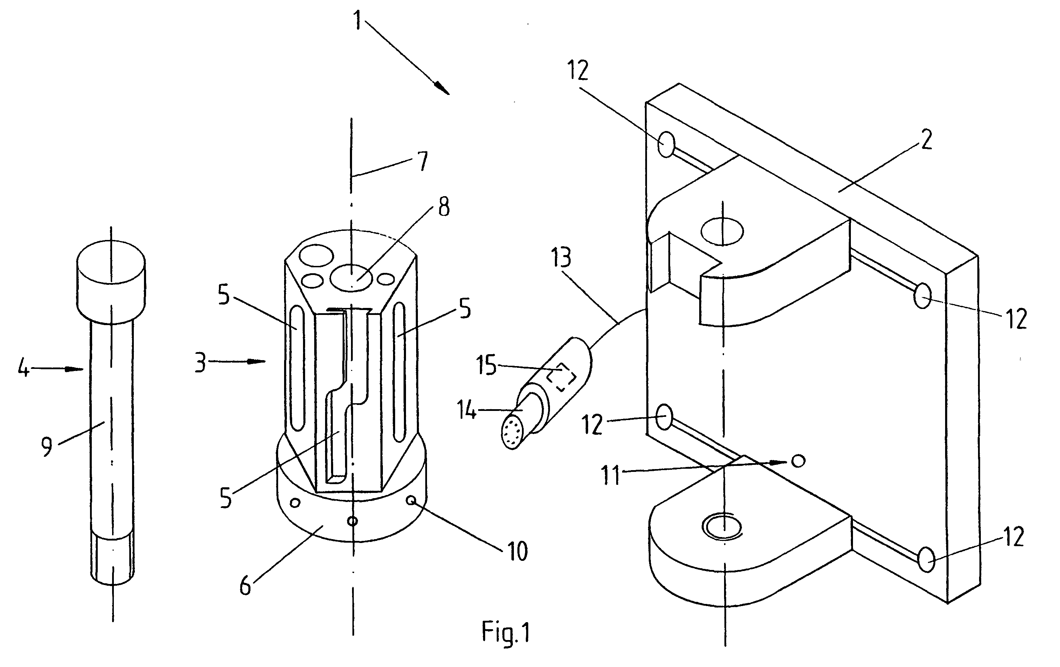

Die in Fig. 1 dargestellte Ausführungsart der erfindungsgemässen Vorrichtung 1 umfasst eine Halterung 2, ein in der Halterung 2 bewegbares Element 3 und ein das bewegbare Element 3 in der Halterung 2 führendes Mittel 4. Das bewegbare Element 3 ist als Zylinder 6 mit einer Zentralachse 7 und einer koaxialen durchgehenden Bohrung 8 gestaltet. Das das bewegbare Element 3 führende Mittel 4 ist ein in der Vorrichtung lösbar angebrachter durch die Bohrung 8 durchgehender Bolzen 9. Der Durchmesser des Bolzens 9 passt nahezu spielfrei in die Bohrung 8. Damit ist eine exakte Positionierung der chirurgischen Instrumente gewährleistet auch wenn verschiedene bewegbare Elemente 3 in der Halterung 2 ausgetauscht werden. Das bewegbare Element 3 enthält mehrere Hohlräume 5, die zur Zentralachse 7 so gestaltet sind, dass jeweils ein in einen entsprechenden Hohlraum 5 eingeführtes chirurgisches Instrument aufgrund der diesem Instrument entsprechenden Gestalt des jeweiligen Hohlraumes 5 in einer zur Vorrichtung 1 genau definierten Position und Orientierung aufgenommen wird. Das bewegbare Element 3 ist drehbar gelagert. Dadurch wird ermöglicht, dass durch Drehung des bewegbaren Elementes 3 um die Zentralachse 7 jeweils eines von verschiedenen chirurgischen Instrumenten in eine im Raum definierte Position gebracht werden kann. Das bewegbare Element 3 ist an seiner äusseren Mantelfläche mit Vertiefungen 10 versehen, die so zu den Hohlräumen 5 korrespondieren, dass das Federdruckteil 11, das an der Halterung 2 angebracht ist, in einer dieser Vertiefungen 10 einrasten kann und damit den korrespondierenden Hohlraum 5 in einer bezüglich der Position und Orientierung der Vorrichtung 1 genau definierten Lage lösbar fixiert. An der Halterung 2 sind sechs Light Emitting Dioden angebracht. Die von den sechs Light Emitting Dioden (LED) 12 emittierten Lichtwellen werden von mindestens zwei zu einer optischen Koordinatenvermessungseinrichtung (in Fig. 1 nicht dargestellt) gehörenden Sensoren detektiert. Die durch die optischen Sensoren empfangenen Signale lassen sich mittels Laufzeitmessung, Interferometrie oder Videogrammetrie in einem Computersystem so verarbeiten, dass aus den Messwerten die genaue Position und Orientierung der Vorrichtung 1 bestimmt werden kann. Am Verbindungskabel 13 zum Computer ist im Stecker 14 ein die Position und Orientierung der Vorrichtung 1 festhaltender, löschbarer elektronischer Datenspeicher 15 angebracht. In der hier beschriebenen Ausführungsform der erfindungsgemässen Vorrichtung handelt es sich bei dem elektronischen Datenspeicher 15 um ein Erasable Programmable Read-Only Memory (EPROM).

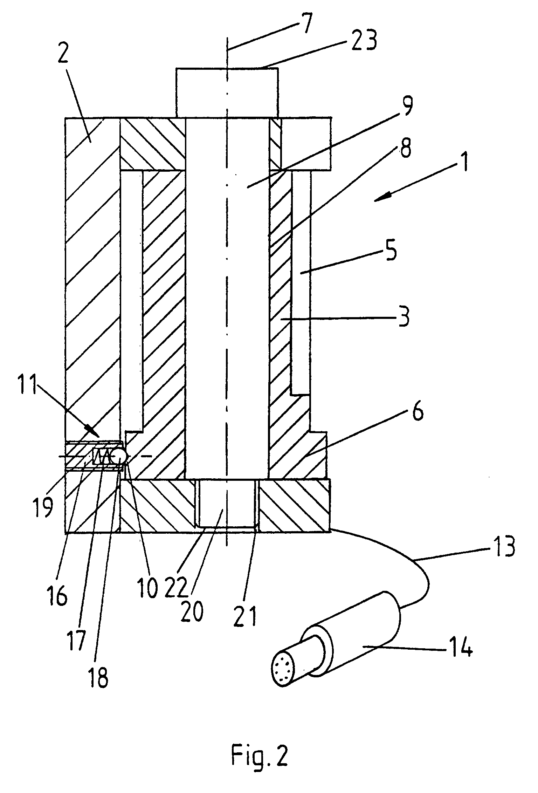

Die in Fig. 2 dargestellte Ausführungsform der erfindungsgemässen Vorrichtung unterscheidet sich von der in Fig. 1 dargestellten Variante nur durch eine Schnittdarstellung des Bolzens 9 und des Arretiermittels 11. Der Bolzen 9 läuft in der Bohrung 8, die das bewegbare Element 3 entlang der Zentralachse 7 vollständig durchdringt. An seinem unteren Ende 22 ist der Bolzen 9 mit einem Absatz 20 mit einem Gewinde zur Befestigung in der Halterung 2 der Vorrichtung 1 versehen. Das obere Ende 23 des Bolzens 9 ist mit Mitteln zur Ein- und Ausdrehung des schraubenartigen Bolzens 9 versehen. Dies kann eine Rändelung, ein Schlitz für einen Schraubenzieher oder ein Innensechskant für einen entsprechenden Schraubenzieher sein. Das bewegbare Element 3 ist an seiner äusseren Mantelfläche mit Vertiefungen 10 versehen. Die Vertiefungen 10 korrespondieren zu den ebenfalls am bewegbaren Element 3 angebrachten Hohlräumen 5 für die Aufnahme der chirurgischen Instrumente. Das in ein entsprechendes Gewinde 19 in der Halterung 2 eingeschraubte Arretiermittel 11 ist bei der hier dargestellten Ausführungsform der erfindungsgemässen Vorrichtung ein Federdruckteil 16. Dieses Federdruckteil 16 besteht im wesentlichen aus einer Zylinderschraube, einer Druckfeder 17 und einer Kugel 18. Die Kugel 18 und die Feder 17 sind in der Zylinderschraube axial zusammenpressbar eingelassen, so dass die Kugel 18 durch die Federkraft in die Vertiefungen 10 einrastet.

## Patentansprüche

1. Vorrichtung (1) zur exakten Kalibrierung und Verfizierung der Genauigkeit von chirurgischen Instrumenten, wobei
die Vorrichtung (1) eine Halterung (2) umfasst, die mit mindestens einem Hohlraum (5) versehen ist, der so gestaltet ist, daß seine Form mit dem darin einführbaren chirurgischen Instrument korrespondiert und damit eine präzise Positionierung des Instrumentes gegenüber der Halterung (2) ermöglicht;
die Vorrichtung (1) mit Mitteln (12) versehen ist, die die Bestimmung der Position und Orientierung der Halterung (2) im Raum ermöglichen;
die Vorrichtung (1) mit einem die Position und Orientierung der Halterung (2) festhaltenden, löschbaren elektronischen Datenspeicher (15) versehen ist,
**dadurch gekennzeichnet, dass** ein in der Halterung (2) bewegbares Element (3) und mindestens ein das bewegbare Element (3) in der Halterung (2) führendes Mittel (4) vorgesehen ist, wobei
das bewegbare Element (3) mit mehreren zur Halterung (2) ausrichtbaren Hohlräumen (5) versehen ist und diese Hohlräume (5) so gestaltet sind, dass sie verschiedene Formen aufweisen, welche zu verschiedenen chirurgischen Instrumenten korrespondieren und damit eine präzise Positionierung der chirurgischen Instrumente gegenüber der Halterung (2) ermöglichen.

2. Vorrichtung nach Anspruch 1, **dadurch gekennzeichnet, dass** das bewegbare Element (3) ein Zylinder (6) mit einer Zentralachse (7) und einer koaxialen durchgehenden Bohrung (8) ist.

3. Vorrichtung nach Anspruch 1 oder 2, **dadurch gekennzeichnet, dass** das führende Mittel (4) ein in der Vorrichtung lösbar angebrachter durch die Bohrung (8) durchgehender Bolzen (9) ist und der Durchmesser des Bolzens (9) nahezu spielfrei in die Bohrung (8) passt.

4. Vorrichtung nach einem der Ansprüche 1 bis 3, **dadurch gekennzeichnet, dass** das bewegbare Element (4) an seiner äusseren Mantelfläche mit Vertiefungen (10) versehen ist, die so zu den Hohlräumen (5) korrespondieren, dass jede Vertiefung (10) durch Arretiermittel (11), die an der Halterung (2) angebracht sind und in diese Vertiefungen (10) lösbar einrasten, einen korrespondierenden Hohlraum (5) in einer bezüglich der Position und Orientierung der Vorrichtung (1) genau definierten Lage fixiert.

5. Vorrichtung nach Anspruch 4, **dadurch gekennzeichnet, dass** das an der Halterung (2) angebrachte, in eine der Vertiefungen (10) am bewegbaren Element (4) einrastende Arretiermittel (11) ein Federdruckteil (16) ist.

6. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, elektromagnetische Wellen emittierende Sender sind.

7. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, optische Leuchtquellen sind.

8. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, optische Reflektoren sind.

9. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, Light Emitting Dioden (LED) sind.

10. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, lntrared Light Emitting Dioden (IRED) sind.

11. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, durch eine Lichtquelle gespiesene optische Faserleiter sind.

12. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, akustische Wellen emittierende Sender sind.

13. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, Mikrophone sind.

14. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, Induktionsspulen sind.

15. Vorrichtung nach einem der Ansprüche 1 bis 5, **dadurch gekennzeichnet, dass** die Mittel (12), die die Bestimmung der Position und Orientierung der Vorrichtung (1) durch eine Vermessungseinrichtung ermöglichen, Hall-Sensoren sind.

16. Vorrichtung nach einem der Ansprüche 1 bis 15, **dadurch gekennzeichnet, dass** das bewegbare Element (3) gegen ein Anderes ausgetauscht werden kann.

17. Vorrichtung nach einem der Ansprüche 1 bis 16, **dadurch gekennzeichnet, dass** der die Position und Orientierung der Halterung (2) festhaltende, löschbare elektronische Datenspeicher (15) ein EPROM (Erasable Programmabel Read-Only Memory) ist.

18. Vorrichtung nach Anspruch 17, **dadurch gekennzeichnet, dass** der elektronische Datenspeicher im Stecker (14) am Verbindungskabel (13) zu einem Computer angebracht ist.

19. Vorrichtung nach einem der Ansprüche 1 bis 17, **dadurch gekennzeichnet, dass** der die Position und Orientierung der Halterung (2) festhaltende, löschbare elektronische Datenspeicher (15) in der Hardware des Computers enthalten ist.

20. Vorrichtung nach einem der Ansprüche 1 bis 16, **dadurch gekennzeichnet, dass** der die Position und Orientierung der Halterung (2) festhaltende, löschbare elektronische Datenspeicher (15) in der Software des Computers programmiert ist.

## Claims

1. Device (1) for gauging and verifying the precision of surgical instruments, whereby
the device (1) encompasses a holder (2), which is provided with at least one cavity (5) being so configured that its contour corresponds to the shape of the respective surgical instrument that can be placed therein, thus permitting precise positioning of the surgical instrument relative to the holder (2);
the device (1) is provided with means (12) which permit the determination of the spatial position and orientation of the holder (2);
the device (1) is equipped with an erasable electronic memory (15) for storing the positional and orientational data of the holder (2),
**characterised in that**
an element (3) that is movable within said holder (2) and at least one medium (4) that guides the movable element (3) within said holder (2) are provided, whereby
the movable element (3) is provided with a plurality of cavities (5) that can be aligned relative to the holder (2), said cavities (5) being so configured that their contours are different in order to correspond to different surgical instruments, thus permitting precise positioning of the surgical instruments relative to the holder (2).

2. Device as in claim 1, **characterized in that** the movable element (3) is a cylinder (6) with a central axis (7) and a coaxial end-to-end bore (8).

3. Device as in claim 1 or 2, **characterized in that** the guiding medium (4) is a bolt (9) detachably mounted in the device and extending through the entire bore (8), said bolt (9) having a diameter that fits with almost no radial clearance into the bore (8).

4. Device as in one of the claims 1 to 3, **characterized in that** the outer surface of the movable element (3) is provided with indentations (10) which correspond to the cavities (5) in such fashion that, by means of detents (11) which are mounted on the holder (2) and which retractably engage in the indentations (10), each such indentation (10) locks a corresponding cavity (5) in a precisely defined location relative to the position and orientation of the device (1).

5. Device as in claim 4, **characterized in that** the detent (11) which is mounted on the holder (2) and engages in one of the indentations (10) on the movable element (3) is a compression-spring-loaded unit (16).

6. Device as in one of the claims 1 to 5, **characterized in that** the means (12) which permit the determination of the location and orientation of the device (1) by a positional measuring system, are transmitters emitting electromagnetic waves.

7. Device as in one of the claims 1 to 5, **characterized in that** the means (12) which permit the determination of the location and orientation of the device (1) by a positional measuring system, are optical light sources.

8. Device as in one of the claims 1 to 5, **characterized in that** the means (12) which permit the determination of the location and orientation of the device (1) by a positional measuring system, are optical light reflectors.

9. Device as in one of the claims 1 to 5, **characterized in that** the means (12) which permit the determination of the location and orientation of the device (1) by a positional measuring system, are light emitting diodes (LEDs).

10. Device as in one of the claims 1 to 5, **characterized in that** the means (12) which permit the determination of the location and orientation of the device (1) by a positional measuring system, are infrared light emitting diodes (IREDs).

11. Device as in one of the claims 1 to 5, **characterized in that** the means (12) which permit the determination of the location and orientation of the device (1) by a positional measuring system, are fiber optics illuminated by a light source.

12. Device as in one of the claims 1 to 5, **characterized in that** the means (12) which permit the determination of the location and orientation of the device (1) by a positional measuring system, are transmitters emitting acoustic waves.

13. Device as in one of the claims 1 to 5, **characterized in that** the means (12) which permit the determination of the location and orientation of the device (1) by a positional measuring system, are microphones.

14. Device as in one of the claims 1 to 5, **characterized in that** the means (12) which permit the determination of the location and orientation of the device (1) by a positional measuring system, are induction coils.

15. Device as in one of the claims 1 to 5, **characterized in that** the means (12) which permit the determination of the location and orientation of the device (1) by a positional measuring system, are Hall-effect detectors.

16. Device as in one of the claims 1 to 15, **characterized in that** the movable element (3) is interchangeable with another movable element.

17. Device as in one of the claims 1 to 16, **characterized in that** the erasable electronic memory (15) storing the positional and orientational data of the holder (2) is an EPROM (erasable programmable read-only memory).

18. Device as in claim 17, **characterized in that** the electronic data storage memory is located in the plug connector (14) of the interconnecting computer cable (13).

19. Device as in one of the claims 1 to 17, **characterized in that** the erasable electronic memory (15) storing the positional and orientational data of the holder (2) is integrated into the computer hardware.

20. Device as in one of the claims 1 to 16, **characterized in that** the erasable electronic memory (15) storing the positional and orientational data of the holder (2) is programmed into the software of the computer.

## Revendications

1. Dispositif (1) pour le calibrage exact et la vérification de la précision d'instruments chirurgicaux, dans lequel
le dispositif (1) comporte un support (2),
le dispositif (1) est pourvu de moyens (12) qui permettent de déterminer la position et d'orienter le support (2) dans l'espace,
le dispositif (1) est pourvu d'une mémoire de données (15) électronique effaçable, enregistrant la position et l'orientation du support (2),
**caractérisé en ce qu'**il est prévu un élément mobile (3) dans le support (2) et au moins un moyen (4) servant à guider dans le support (2) l'élément mobile (3) qui est pourvu d'au moins un espace creux (5) qui est réalisé de telle manière que sa forme correspond avec l'instrument chirurgical introduit dans celui-ci et permet ainsi un positionnement précis de l'instrument par rapport au support (2),
l'élément mobile (3) étant pourvu de plusieurs espaces creux (5) orientables par rapport au support (2) et ces espaces creux (5) étant réalisés de telle manière qu'ils présentent différentes formes qui correspondent aux différents instruments chirurgicaux et permettent ainsi un positionnement précis des instruments chirurgicaux par rapport au support (2).

2. Dispositif selon la revendication 1, **caractérisé en ce que** l'élément mobile (3) est un cylindre (6) avec un axe central (7) et un orifice (8) coaxial traversant.

3. Dispositif selon la revendication 1 ou 2, **caractérisé en ce que** l'élément guide (4) est un goujon (9) traversant l'orifice (8) placé de manière mobile dans le dispositif, et le diamètre du goujon (9) s'adapte pratiquement sans jeu dans l'orifice (8).

4. Dispositif selon une des revendications 1 à 3, **caractérisé en ce que** l'élément mobile (3) est pourvu sur sa surface d'enveloppe extérieure d'encoches (10) qui correspondent aux espaces creux (5), **en ce que** chaque encoche (10) fixe un espace creux (5) correspondant dans une position précisément définie en ce qui concerne la position et l'orientation du dispositif (1) grâce aux moyens de blocage (11) qui sont placés sur le support (2) et s'encliquètent de manière mobile dans ces encoches (10).

5. Dispositif selon la revendication 4, **caractérisé en ce que** le moyen de blocage (11) placé sur le support (2), s'encliquetant dans une des encoches (10) sur l'élément mobile (3) est un ressort de pression (16).

6. Dispositif selon une des revendications 1 à 5, **caractérisé en ce que** les moyens (12) qui permettent de déterminer la position et l'orientation du dispositif (1) grâce à un dispositif de mesure sont des émetteurs émettant des ondes électromagnétiques.

7. Dispositif selon une des revendications 1 à 5, **caractérisé en ce que** les moyens (12) qui permettent de déterminer la position et l'orientation du dispositif (1) grâce à un dispositif de mesure sont des sources lumineuses optiques.

8. Dispositif selon une des revendications 1 à 5, **caractérisé en ce que** les moyens (12) qui permettent de déterminer la position et l'orientation du dispositif (1) grâce à un dispositif de mesure sont des réflecteurs optiques.

9. Dispositif selon une des revendications 1 à 5, **caractérisé en ce que** les moyens (12) qui permettent de déterminer la position et l'orientation du dispositif (1) grâce à un dispositif de mesure sont des diodes luminescentes (LED)

10. Dispositif selon une des revendications 1 à 5, **caractérisé en ce que** les moyens (12) qui permettent de déterminer la position et l'orientation du dispositif (1) grâce à un dispositif de mesure sont des diodes luminescentes infrarouges (IRED).

11. Dispositif selon une des revendications 1 à 5, **caractérisé en ce que** les moyens (12) qui permettent de déterminer la position et l'orientation du dispositif (1) grâce à un dispositif de mesure sont des conducteurs par fibres optiques alimentés par une source lumineuse.

12. Dispositif selon une des revendications 1 à 5, **caractérisé en ce que** les moyens (12) qui permettent de déterminer la position et l'orientation du dispositif (1) grâce à un dispositif de mesure sont des émetteurs émettant des ondes acoustiques.

13. Dispositif selon une des revendications 1 à 5, **caractérisé en ce que** les moyens (12) qui permettent de déterminer la position et l'orientation du dispositif (1) grâce à un dispositif de mesure sont des microphones.

14. Dispositif selon une des revendications 1 à 5, **caractérisé en ce que** les moyens (12) qui permettent de déterminer la position et l'orientation du dispositif (1) grâce à un dispositif de mesure sont des bobines d'induction.

15. Dispositif selon une des revendications 1 à 5, **caractérisé en ce que** les moyens (12) qui permettent de déterminer la position et l'orientation du dispositif (1) grâce à un dispositif de mesure sont des capteurs de Hall.

16. Dispositif selon une des revendications 1 à 15, **caractérisé en ce que** l'élément mobile (3) peut être remplacé par un autre.

17. Dispositif selon une des revendications 1 à 16, **caractérisé en ce que** la mémoire de données (15) électronique effaçable, enregistrant la disposition et l'orientation du support (2) est une mémoire EPROM (mémoire morte effaçable et programmable).

18. Dispositif selon la revendication 17, **caractérisé en ce que** la mémoire de données électronique est placée dans le connecteur (14) sur le câble de connexion (13) à un ordinateur.

19. Dispositif selon une des revendications 1 à 17, **caractérisé en ce que** la mémoire de données (15) électronique effaçable, enregistrant la disposition et l'orientation du support (2) est contenue dans le matériel de l'ordinateur.

20. Dispositif selon une des revendications 1 à 16, **caractérisé en ce que** la mémoire de données (15) électronique effaçable, enregistrant la disposition et l'orientation du support (2) est programmée dans le logiciel de l'ordinateur.
